# EUROPEAN PATENT SPECIFICATION

(11) **EP 2 509 591 B1**
(45) Date of publication and mention of the grant of the patent: **25.11.2015**
(21) Application number: 10829435.6
(22) Date of filing: 25.10.2010
(51) Int. Cl.: A61K 31/277, A61P 1/00, A61P 1/12, A61P 1/18, A61P 3/04, A61P 3/06, A61P 3/10, A61P 9/02, A61P 17/00, A61P 17/06, A61P 25/06, A61P 25/14, A61P 25/16

(54) **METHODS AND COMPOSITIONS FOR TREATING DISEASE OR CONDITION RELATED TO OREXIN RECEPTOR 1, OREXIN RECEPTOR 2, SOMATOSTATIN RECEPTOR 2 OR DOPAMINE D2L RECEPTOR**
VERFAHREN UND ZUSAMMENSETZUNGEN ZUR BEHANDLUNG VON ERKRANKUNGEN ODER LEIDEN IN ZUSAMMENHANG MIT DEM OREXIN-REZEPTOR 1, DEM OREXIN-REZEPTOR 2, DEM SOMATOSTATIN-REZEPTOR 2 ODER DEM DOPAMIN-D2L-REZEPTOR
MÉTHODES ET COMPOSITIONS DESTINÉES AU TRAITEMENT DE MALADIES OU D'AFFECTIONS ASSOCIÉES AU RÉCEPTEUR 1 DE L'OREXINE, AU RÉCEPTEUR 2 DE L'OREXINE, AU RÉCEPTEUR 2 DE LA SOMATOSTATINE OU AU RÉCEPTEUR D2L DE LA DOPAMINE

(30) Priority: 10.11.2009 US 259688 P
(43) Date of publication of application: 17.10.2012
(73) Proprietor: Center Laboratories, Inc., Taipei City 105, Taiwan (TW)
(72) Inventor: LEE, Huai-Cheng, Taiwan (CN); SHANE, Guang-tzuu, Taiwan (CN); WANG, Hsi-chieh, Taiwan (CN); LIN, Rongjin, Taiwan (CN)
(74) Representative: Lang, Christian
(86) International application number: PCT/CN2010/001681
(87) International publication number: WO 2011/057471

(56) References cited:
- WO-A2-2009/090453
- WO-A2-2009/090453
- CN-A- 1 262 098
- US-A- 4 788 219
- US-A- 5 525 601
- US-A1- 2002 037 843
- US-A1- 2008 045 603
- DATABASE WPI Week 200062 Thomson Scientific, London, GB; AN 2000-639081 XP002699820, & CN 1 262 098 A (YANG Y) 9 August 2000 (2000-08-09)
- BALON R ET AL: "Calcium channel blockers for anxiety disorders?", ANNALS OF CLINICAL PSYCHIATRY, ELSEVIER, US, vol. 8, no. 4, December 1996 (1996-12), pages 215-220, XP009103793, ISSN: 1040-1237, DOI: 10.3109/10401239609147764
- RAEBURN D ET AL: "CNS disorders and calcium antagonists", TRENDS IN PHARMACOLOGICAL SCIENCES, ELSEVIER, HAYWARTH, GB, vol. 9, no. 4, April 1988 (1988-04), pages 117-119, XP025843170, ISSN: 0165-6147, DOI: 10.1016/0165-6147(88)90186-1 [retrieved on 1988-04-01]
- MARKLEY ET AL: "Verapamil and migraine prophylaxis: mechanisms and efficacy", AMERICAN JOURNAL OF MEDICINE, EXCERPTA MEDICA, INC, UNITED STATES, vol. 90, no. 5, 17 May 1991 (1991-05-17), pages S48-S53, XP023307989, ISSN: 0002-9343, DOI: 10.1016/0002-9343(91)90486-H [retrieved on 1991-05-17]
- IVKOVIC M ET AL: "The influence of verapamil on cognitive functions in experimental model of Alzheimer's disease in rats", EUROPEAN NEUROPSYCHOPHARMACOLOGY, ELSEVIER SCIENCE PUBLISHERS BV, AMSTERDAM, NL, vol. 8, November 1998 (1998-11), pages S280-S281, XP027369888, ISSN: 0924-977X [retrieved on 1998-11-01]
- DATABASE MEDLINE [Online] US NATIONAL LIBRARY OF MEDICINE (NLM), BETHESDA, MD, US; November 1997 (1997-11), POPOVIC M ET AL: "Neuroprotective effect of chronic verapamil treatment on cognitive and noncognitive deficits in an experimental Alzheimer's disease in rats.", XP002699821, Database accession no. NLM9522258 & THE INTERNATIONAL JOURNAL OF NEUROSCIENCE NOV 1997, vol. 92, no. 1-2, November 1997 (1997-11), pages 79-93, ISSN: 0020-7454
- DATABASE MEDLINE [Online] US NATIONAL LIBRARY OF MEDICINE (NLM), BETHESDA, MD, US; November 1986 (1986-11), WALSH T L ET AL: "Calcium antagonists in the treatment of Tourette's disorder.", XP002699822, Database accession no. NLM3465244 & THE AMERICAN JOURNAL OF PSYCHIATRY NOV 1986, vol. 143, no. 11, November 1986 (1986-11), pages 1467-1468, ISSN: 0002-953X
- UHR S B ET AL: "Effects of verapamil administration on negative symptoms of chronic schizophrenia.", PSYCHIATRY RESEARCH MAR 1988, vol. 23, no. 3, March 1988 (1988-03), pages 351-352, XP002699823, ISSN: 0165-1781
- DANG YUELAN: 'Isoptin analgesic action and effect on morphine dependence' CHINESE BULLETIN ON DRUG DEPENDENCE vol. 5, no. 3, 1996, CHINESE, pages 163 - 166, XP008158996
- WU CHENGHAN ET AL.: 'Observation of Effect on Prevention and Treatment of Migraine with Verapamil.' ZONGHE LINCHUANG YIXUE (CHINESE) vol. 7, no. 4, 1991, pages 192 - 193, XP008155781
- JIN QIAN ET AL.: 'Inhibitive efficacy of verapamil on Lewis lung cancer and its effect on the expression of MMP-9.' CHINESE JOURNAL OF CLINICAL PHARMACY vol. 15, no. 5, 2006, CHINESE, pages 267 - 270, XP008156589
- LIU SHUNYING ET AL.: 'Reversal Effects of Verapamil on Adriamycm Resistance of Human Liver Cancer Cells.' JIANGSU PHARMACEUTICAL AND CLINICAL RESEARCH vol. 7, no. 4, 1999, CHINESE, pages 5 - 7, XP008155786
- SHAO NINGSHENG ET AL.: 'The Effect of Verapamil on L1210 Leukemia Cells in Vitro.' JOURNAL OF LANZHOU MEDICAL COLLEGE vol. 16, no. 2, 1990, CHINESE, pages 78 - 80, XP008155874
- PU QINGFAN ET AL.: 'Therapeutic Effects of Continous Regional Arterial Infusion with Verapamil on Progression of Acute Pancreatitis.' CHINESE JOURNAL OF BASES AND CLINICS IN GENERAL SURGERY vol. 14, no. 2, March 2007, CHINESE, pages 200 - 202, XP008156042
- YANG YAN ET AL.: 'The effect of cyclosporin A and verapamil on psoriasis in experimental animal models' CHINESE JOURNAL OF CLINICAL PHARMACOLOGY AND THERAPEUTICS vol. 2, no. 2, 1997, CHINESE, pages 95 - 97, XP008156606
- ZHANG HONGTAO ET AL.: 'Study of effects of Verapamil on growth of meningioma.' CHINA JOURNAL OF MODERN MEDICINE vol. 15, no. 6, March 2005, pages 811 - 814, XP008156040
- WANG ZHAOJUN ET AL.: 'The Effects of Verapamil on Plasma Fibrinogen and Restenosis after Angioplasty in Rabbits.' JOURNAL OF ZHENJIANG MEDICAL COLLEGE vol. 11, no. 3, 2001, CHINESE, pages 294 - 297, XP008156588
- LIU HAILIN ET AL.: 'Effects of verapamil and nifedipine on the proliferation, collagen and hyaluronic acid synthesis of human lung fibroblasts.' CHINESE PHARMACOLOGICAL BULLETIN vol. 12, no. 5, October 1996, CHINESE, pages 456 - 458, XP008155485
- ZHOU CHENGMIN.: 'The toxic reaction of isoptin.' CHINESE JOURNAL OF HOSPITAL PHARMACY vol. 7, no. 3, 1987, CHINESE, pages 105 - 107, XP008155782

## Description

### TECHNICAL FIELD

This disclosure in general relates to the field of pharmacology.

### BACKGROUND ART

### Description of Related Art

Objectives of this disclosure are identification of compounds that may bind to endogenous neurotransmitter receptors. Accordingly, verapamil and their stereoisomers, metabolites, derivatives, salts, solvates and combinations thereof are identified and formulated into a pharmaceutical composition for treating a disease or condition related to orexin receptor 1 , orexin receptor 2, somatostatin receptor 2 or dopamine D_{2L} receptor.

WO 2009/090453 A2 discloses the use of (R)-verapamil for treating, among others; migraine headaches, cluster headaches, depression, alcoholism, generalized anxiety disorder, prostatic hyperplasia (cancer), IBD and sleep disorders. US 2008/045603 A1 discloses the use of (R)-verapamil for treating, among others; migraine headaches, cluster headaches, depression, alcoholism, generalized anxiety disorder, prostatic hyperplasia (cancer) and sleep disorders. US 5,525,601 discloses the use of verapamil for treating ocular pain. CN 1 262 098 A discloses the use of verapamil for treating heroin or opium addiction. US 4,788,219 A discloses the use of verapamil for reducing cancer metastasis, e.g. metastasis of melanoma and colon cancer cells. Balon R. et. al ("Calcium channel blockers for anxiety disorders?", ANNNALS OF CLINICAL PSYCHIATRY, vol. 8, no. 3, 1 December 1996, pages 215-220) disclose the possible beneficial use of verapamil for treating anxiety disorders, indicating the previously reported beneficial use of verapamil to treat mood disorders, IBD, schizophrenia, tardive dyskinesia and Tourette's syndrome. Raeburn D. et al. ("CNS disorders and calcium antagonists", TRENDS IN PHARMACOLOGICAL SCIENCES, vol. 9, no. 4, 1 April 1988, pages 117-119) report the beneficial use of verapamil in schizophrenia and the control of pain. Markley et al. ("Verapamil and migraine prophylaxis: mechanisms and efficacy", AMERICAN JOURNAL OF MEDICINE, vol. 90, no. 5, 17 May 1991, pages S48-S53) report the beneficial effects of verapamil in the treatment of migraine and cluster headache. Ivkovic M. et al. ("The influence of verapamil on cognitive functions in experimental model of Alzheimer's disease in rats", EUROPEAN NEUROPSYCHOPHARMACOLOGY, vol. 8, 1 November 1998, pages S280-S281) and Popovic M. et al. ("Neuroprotective effect of chronic verapamil Alzheimer's disease in rats" INTERNATIONAL JOURNAL OF NEUROSCIENCE November 1997, vol. 92, no. 1-2, November 1997, pages 79-93) teach the ability of verapamil to improve learning cognitive and behavioural deficits in experimental animal models of Alzheimer's disesase. US 2002/037843 A1 discloses the use of verapamil to treat Alzhjeimer's disease through its ability to act as a ATP binding cassette (ABC) transporter blocker. Walsh T. L. et al. ("Calcium antagonists in the treatment of Tourette's disorder", THE AMERICAN JOURNAL OF PSYCHIATRY November 1986, vol. 143, no. 11, November 1986, pages 1467-1468) teach positive effects of verapamil in the treatment of Alzheimer's and Tourette's syndrome.

### SUMMARY

The invention is directed to verapamil for use in the treatment of a subject suffering from obesity or diabetes, wherein the verapamil is capable of antagonizing the function of orexin receptor 1 or orexin receptor 2 of the subject.

As embodied and broadly described herein, disclosure herein features methods and composition for treating a disorder related to orexin receptor 1 , orexin receptor 2, somatostatin receptor 2 or dopamine D2L receptor.

Therefore, it is disclosed a method of binding at least one of orexin receptor 1 , orexin receptor 2, somatostatin receptor 2 or dopamine D2L receptor in a subject, comprising the step of administering verapamil to the subject In some embodiments, the verapamil is any of (R)-(+)-verapamil, (S)-(-)-verapamil), a mixture of (R)-(+)-verapamil and (S)-(-)-verapamil, a pharmaceutically acceptable derivative, stereoisomer, metabolite, salt or solvate thereof, or a combination thereof. In one specific example, the verapamil is a racemic mixture of (R)-(+)-verapamil and (S)-(-)-verapamil. It is found that both (R)-(+)-verapamil hydrochloride an (S)-(-)-verapamil hydrochloride act as agonists to somatostatin receptor 2, and antagonists to orexin receptor 1, orexin receptor 2 or dopamine (D2L) receptor.

It is further disclosed a method of treating a subject having a disease or condition related to orexin receptor 1 , orexin receptor 2, somatostatin receptor 2 or dopamine D2i_ receptor, comprising the step of administering an effective amount of verapamil and a pharmaceutically acceptable excipient to the subject, wherein the verapamil is any of (R)-(+)-verapamil, (S)-(-)-verapamil, a mixture of (R)-(+)-verapamil and (S)-(-)-verapamil, a pharmaceutically acceptable derivative, stereoisomer, metabolite, salt or solvate thereof, or a combination thereof. The verapamil is (R)-(+)-verapamil, a pharmaceutically acceptable derivative, stereoisomer, metabolite, salt or solvate thereof, or a combination thereof.

The disease or condition related to orexin receptor 1 or orexin receptor 2 is selected from the group consisting of obesity, migraine, cluster headache, narcolepsy, Parkinson's disease, Alzheimer's disease, depression, addictions, anxiety, cancer, diabetes, insomnia, irritable bowel syndrome, neuropathic pain, pain, schizophrenia, sleep disorder, and Tourette syndrome. The disease or condition related to somatostatin receptor 2 is selected from the group consisting of Crushing's syndrome, gonadotropinoma, gastrinoma, Zollinger-Ellison syndrome, hypersecretory diarrhea related to AIDS and other conditions, irritable bowel syndrome, pancreatitis, Crohn's disease, systemic sclerosis, thyroid cancer, psoriasis, hypotension, panic attacks, scleroderma, small bowel obstruction, gastroesophageal reflux, duodenogastric reflux, Grave's disease, polycystic ovary disease, upper gastrointestinal bleeding, pancreatic pseudocyst, pancreatic ascites, leukemia, meningioma, cancer cachexia, acromegaly, restenosis, hepatoma, lung cancer, melanoma, wasting, type 2 diabetes, Syndrome X, fibrosis, hyperlipidemia, hyperamylinemia, hyperprolactinemia, prolactinomas, cluster headache, depression, neuropathic pain and pain. The disease or condition related to dopamine D_{2L} receptor is selected from the group consisting of schizophrenia, anxiety, depression, migraine pain, Parkinson's disease, addiction and Tourette syndrome.

It is further disclosed a pharmaceutical composition for treating a disease or condition related to orexin receptor 1 , orexin receptor 2, somatostatin receptor 2 or dopamine D_{2L} receptor in a subject, comprising verapamil and a pharmaceutically acceptable excipient, wherein the verapamil is any of (R)-(+)-verapamil, (S)-(-)-verapamil, a mixture of (R)-(+)-verapamil and (S)-(-)-verapamil, a pharmaceutically acceptable derivative, stereoisomer, metabolite, salt or solvate thereof, or a combination thereof. The verapamil is (R)-(+)-verapamil, a pharmaceutically acceptable derivative, stereoisomer, metabolite, salt or solvate thereof, or a combination thereof..

Further details are set forth in the accompanying description below. The features of the invention are defined in the accompanying claims 1 to 3.

It is to be understood that both the foregoing general description and the following detailed description are intended to provide further explanation.

It is hereinafter described in detail with respect to methods and compositions for treating a disease or condition related to orexin receptor 1 , orexin receptor 2, somatostatin receptor 2 or dopamine D_{2L} receptor in a subject.

Verapamil is capable of binding to orexin receptor 1, orexin receptor 2, somatostatin receptor 2 or dopamine D_{2L} receptor. Specifically, verapamil act as an agonist to somatostatin receptor 2; and an antagonist to orexin receptor 1 , orexin receptor 2 or dopamine D_{2L} receptor. Accordingly, a method of binding at least one receptor selected from the group consisting of orexin receptor 1 , orexin receptor 2, somatostatin receptor 2 or dopamine D2i receptor in a subject, comprises administering verapamil to the subject.

The term "verapamil (2-(3,4-dimethoxyphenyl)-5-{[2-(3,4-dimethoxyphenyl)ethyl]-(methyl) amino}-2-prop-2-ylpentanenitrile)" herein refers to verapamil, its stereoisomers, derivatives, metabolites, salts, solvates and/or combinations thereof, and may be any of (R)-(+)-verapamil, (S)-(-)-verapamil, a mixture of (R)-(+)-verapamil and (S)-(-)-verapamil, a pharmaceutically acceptable derivative, stereoisomer, metabolite, salt or solvate thereof, or a combination thereof. Verapamil is known to exist in either (R)- or (S)-form. It is well known that pharmacodynamics and pharmacokinetics of the (R)-(+)-verapamil and (S)-(-)-verapamil differs in vivo, with (S)-form more potent than the (R)-form. In one embodiment of this disclosure, verapamil is provided in (R)-form. In another example, verapamil is provided in (S)-form. In some examples, verapamil is proved as a mixture of (R)-(+)-verapamil and (S)-(-)-verapamil, for example, about 90% (R)-form and about 10% (S)-form, about 80% (R)-form and about 20% (S)-form, about 70% (R)-form and about 30% (S)-form, about 60% (R)-form and about 40% (S)-form, about 40% (R)-form and about 60% (S)-form, about 30% (R)-form and about 70% (S)-form, about 20% (R)-form and about 80% (S)-fom, or about 10% (R)-form and about 90% (S)-form. In one specific example, verapamil exists as a racemic mixture containing approximately equal amount of (R)-(+)-verapamil and (8)-(-)-verapamil, that is, with (R)- and (S)-form being about 50%, respectively.

The term "a salt" refers herein a salt which is formed by the interaction of a base (such as verapamil in this disclosure) with an acid, including organic or inorganic types of acids such as hydrochloric acid, hydrobromic acid, hydroiodic acid, perchloric acid, sulfuric acid, nitric acid, phosphoric acid, propionic acid, glycolic acid, acetic acid, pyruvic acid, malonic acid, succinic acid, maleic acid, fumaric acid, tartaric acid, citric acid, benzoic acid, carbonic acid, cinnamic acid, methanesulfonic acid, ethanesulfonic acid, hydroxyethanesulfonic acid, benzenesulfonic acid, p-toluene sulfonic acid, cyclohexanesulfamic acid, salicyclic acid, p-aminosalicyclic acid, 2-phenoxybenzoic acid and 2-acetoxybenzoic acid. In one preferred example, the salt is verapamil hydrochloride. The term "solvate" herein refers to a complex formed by the interaction of a compound (such as verapamil in this disclosure) with surrounding solvent molecules, such as water, ethanol, and etc. In one example, the solvate is verapamil hydrochloride hydrate.

The term "racemic" as used herein refers to a mixture of the enantiomers, or stereoisomers, of a pharmaceutically active agent, in which neither enantiomer or stereoisomer is substantially purified from the other.

In another aspect of the present disclosure, a method of treating a disease or condition related to orexin receptor 1 , orexin receptor 2, somatostatin receptor 2 or dopamine D_{2L} receptor, comprises administering to a subject an effective amount of verapamil and a pharmaceutically acceptable excipient.

As described above, verapamil may be any of (R)-(+)-verapamil, (S)-(-)-verapamil, a mixture of (R)-(+)-verapamil and (S)-(-)-verapamil, a pharmaceutically acceptable derivative, stereoisomer, metabolite, salt or solvate thereof, or a combination thereof.

The term "pharmaceutically acceptable excipient" refers to compounds that are compatible with other ingredients in a pharmaceutical composition and would not endanger the subject after administering.

The term "agonist" refers herein agents or drugs that upon binding to their receptors elicit or trigger a biological response.

The term "antagonist" refers herein agents or drugs that neutralize or impede the action or effects of others, e.g., a drug that binds to a receptor without eliciting a biological response and effectively blocking the binding of a substance that could elicit such a response.

The disease or condition related to orexin receptor 1 or 2 is selected from the group consisting of obesity, migraine, cluster headache, Parkinson's disease, Alzheimer's disease, depression, addictions, anxiety, cancer, diabetes, insomnia, irritable bowel syndrome, narcolepsy, neuropathic pain, pain, schizophrenia, sleep disorder, and Tourette syndrome. In other embodiments, the disease or condition related to somatostatin 2 receptor is selected from the group consisting of Crushing's syndrome, gonadotropinoma, gastrinoma, Zollinger-Ellison syndrome, hypersecretory diarrhea related to AIDS and other conditions, irritable bowel syndrome, pancreatitis, Crohn's disease, systemic sclerosis, thyroid cancer, psoriasis, hypotension, panic attacks, scleroderma, small bowel obstruction, gastroesophageal reflux, duodenogastric reflux, Grave's disease, polycystic ovary disease, upper gastrointestinal bleeding, pancreatic pseudocyst, pancreatic ascites, leukemia, meningioma, cancer cachexia, acromegaly, restenosis, hepatoma, lung cancer, melanoma, wasting, type 2 diabetes, Syndrome X, fibrosis, hyperlipidemia, hyperamylinemia, hyperprolactinemia, prolactinomas, cluster headache, depression, neuropathic pain and pain. In still some embodiments, the disease or condition related to dopamine D2i_ receptor is selected from the group consisting of schizophrenia, anxiety, depression, migraine, pain, Pakinson's disease, Tourette syndrome and addicition.

Thus, it is a further aspect of this disclosure to describe a pharmaceutical composition for treating a disease or condition related to orexin receptor 1 , orexin receptor 2, somatostatin receptor 2 or dopamine D_{2L} receptor in a subject, comprising a therapeutically effective amount of verapamil and a pharmaceutically acceptable excipient. The verapamil, the pharmaceutically acceptable excipient and the disease or condition related to any of the receptor are as described above.

The pharmaceutical composition of this disclosure may be administered by oral, parenteral (e.g., intramuscular, intraperitoneal, intravenous or subcutaneous injection or implant), nasal, sublingual, topical or transdermal routes and can be formulated into various dosage forms suitable for each route of administration. A number of suitable dosage forms are described below, but are not meant to include all possible choices. One of skilled person in the art is familiar with the various dosage forms that are suitable for use in each route. It is to be noted that the most suitable route in any given case would depend on the nature or severity of the disease or condition being treated. The active ingredient, such as verapamil, is mixed with at least one pharmaceutically acceptable excipient including, but are not limited to, fillers, binders, lubricants, glidants, disintegrants and the like. Suitable fillers may include one or more of lactose, microcrystalline cellulose, polyethylene glycols, mannitol, calcium phosphate, calcium sulfate, kaolin, dry starch, sorbitol, powdered sugar, prosolv, and the like. Suitable disintegrants may include one or more of starch, croscarmellose sodium, crospovidone, sodium starch glycolate, hydroxypropylcellulose, and the like. Suitable glidants may include one or more of colloidal silicon dioxide, talc or cornstarch, and the like. Suitable lubricants comprises one or more of hydrogenated vegetable oil, hydrogenated castor oil, light mineral oil, glycerol monostearate, glycerol monobehenate, glyceryl behenate, glyceryl palmitostearate, and the like.

In some embodiments, the pharmaceutical compositions of this disclosure are solid dosage forms for oral administeration. Such solid dosage forms may be capsules, sachets, tablets, pills, lozengens, powders or granules. In such forms, the active ingredient such as verapamil is mixed with at least one pharmaceutically acceptable excipient as described above. Any of the described solid dosage forms may optionally contain coatings and shells, such as enteric coatings, and coatings for modifying the release rate of any of the ingredients. Examples of such coatings are well known in the art. In one example, the pharmaceutical compositions of this disclosure are tablets such as quick-release tablets. In still another example, the pharmaceutical compositions of this disclosure are formulated into sustained release forms. In another example, the pharmaceutical compositions of this disclosure are powders that are encapsulated in soft and hard gelatin capsules.

In some embodiments, the pharmaceutical compositions of this disclosure are liquid dosage forms for oral administration. The liquid formulation may further include a buffering agent to maintain a desired pH. The liquid dosage formulations may also be filled into soft gelatin capsules. For example, the liquid may include a solution, suspension, emulsion, microemulsion, precipitate or any desired liquid media carrying (R)-(+)-verapamil, (S)-(-)-verapamil, a mixture of (R)-(+)-verapamil and (S)-(-)-verapamil, a pharmaceutically acceptable derivative, stereoisomer, metabolite, salt or solvate thereof, or a combination thereof. The liquid may be designed to improve the solubility of (R)-(+)-verapamil, (S)-(-)-verapamil, a mixture of (R)-(+)-verapamil and (S)-(-)-verapamil, a pharmaceutically acceptable derivative, stereoisomer, metabolite, salt or solvate thereof, or a combination thereof to form a drug-containing emulsion or disperse phase upon release. In such forms, the active ingredient is mixed with at least one pharmaceutically acceptable excipient as described above.

In some embodiments, the pharmaceutical compositions of this disclosure are formulations suitable for parenteral administration, such as administration by injection, which includes, but is not limited to, subcutaneous, bolus injection, intramuscular, intraperitoneal and intravenous injection. The pharmaceutical compositions may be formulated as isotonic suspensions, solutions or emulsions in oily or aqueous vehicles, and may contain formulatoray agents such as suspending, stabilizing or dispersing agents. Alternatively, the compositions may be provided in dry form such as powders, crystallines or freeze-drized solids with sterile pyrogen-free water or isotonic saline before use. They may be presented in sterile ampoules or vials. In such forms, the active ingredient, such as verapamil, is mixed with at least one pharmaceutically acceptable excipient as described above.

In some embodiments, the pharmaceutical compositions of this disclosure are formulations suitable for intranasal administration or administration by inhalation. The compositons are delivered in the form of a solution or suspension from a pump spray container that is squeened or pumped by the patient or in the form of a dry powder or as an aerosol spray from a pressurized container or a nebuilizer, with the use of a suitable propellant such as dichlorodifluoromethane, trichlorofluoromethane, dichlorotetrafluoroethane, a hydrofluoroalkane such as 1,1,1,2-tetrafluoroethane (HFA 134A^{™}) or 1112333-heptafluoropropane (HFA 227EA^{™}), carbon dioxide or other suitable gas. In the case of a pressurized aerosol, the dosage unit may be determined by providing a valve to deliver a metered amount. The pressurized container or nebuilizer may contain a solution or suspension of the composition of this disclosure. Capsules or cartridges for use in an inhaler or insufflators may be formulated to contain a powder mix of the active compound of this disclosure such as (R)-(+)-verapamil, (S)-(-)-verapamil, a mixture of (R)-(+)-verapamil and (S)-(-)-verapamll, a pharmaceutically acceptable derivative, stereoisomer, metabolite, salt or solvate thereof, or a combination thereof. In such forms, the active ingredient is mixed with at least one pharmaceutically acceptable excipient as described above.

In some embodiments, the pharmaceutical compositions of this disclosure are dosage formulations that are suitable for topical or transdermal administration. Such formulations include sprays, ointments, pastes, creams, lotions, gels, solutions and patches. Such formulations may optionally include excipients such as animal and vegetable fats, oils, waxes, paraffins, starch, tragacanth, cellulose derivatives, silicons, bentonites, silicic acid, talc, zinc oxide or mixtures thereof. Sprays may also include excipients such as talc, silicic acid, aluminum hydroxide and calcium silicate; additionally, sprays may contain propellants such as chlorofluoro-hydrocarbons and volatile hydrocarbons such as butane and propane. Transdermal patches may be made by dissolving, dispersing or incorporating a pharmaceutical composition of the present disclosure in a suitable medium, such as elastomeric matrix material. Absorption enhancers may also be used to increase the flux of the mixture across the skin. Alternatively, the composition of the present disclosure may be formulated into a lotion or a cream. In such forms, the active ingredient, such as verapamil is mixed with at least one pharmaceutically acceptable excipient as described above.

It must be noted that as used herein and in the appended claims, the singular forms "a", "an", and "the" include plural referents unless the context clearly dictates otherwise.

Unless defined otherwise, all technical and scientific terms used herein have the same meaning as commonly understood by one of ordinary skill in the art. The following examples are described for illustrative purposes.

### EXAMPLES

### BINDING AFFINITY ASSAY

(R)-(+)-verapamil hydrochloride and (S)-(-)-verapamil hydrochloride were obtained from Sigma-Aldrich<(R)> (USA). Binding activity was evaluated to assess the selectivity of verapamil on various receptors. The binding activity of the reported receptors was analyzed using the following known methods. Primary screening were performed in duplicate and presented in semi-quantitative data (e.g., IC5o, K, and nH). Where presented, IC50 values were determined by a non-linear, least squares regression analysis using MathIQ(TM) (ID Bussiness Solutions Ltd., UK). Where inhibition constants (K,) presented, the K, value were calculated using the equation of Cheng and Prusoff (Cheng. Y., Prusoff, W.H., (1973) Biochem. Pharmacol. 22:3099-3108) using the observed IC5o of the tested compound, the concentration of radioligand employed in the assay and the historical values of the KQ of the ligand. Where presented, the Hill coefficient ([Pi][Eta]), defining the slope of the competitive binding curve, was calculated using MathIQ(TM).

Orexin receptor 1 or 2 binding affinity was examined using the methods disclosed by Kukkonen et al., (2002) Am J Physiol Cell Physiol 283:C1567-C1591 ; and Lanfmead et al., (2004) Br J Pharmacol 141 (2):340-346. Calcium channel (L-type) binding activity was examined using the methods disclosed by Church and Zsoter, (1980) Can J Physiol. Pharmacol. 58:254-264; and Spedding. (1984) M. Eur J Pharmacol, 83:21 1. Somaostatin receptor 2 binding activity was estimated by the method described by Feniuk et al., (1993) Br J Pharmacol. 1 10:1 156-1 164. Adensoine receptor binding activity was examined uisng the method described by Varani et al., (1996) Br J Pharmacol. 1 17:1693-1701. Bradykinin receptor binding activity was examined uisng the methods described by Horlick et al., (1999) Immunopharmacology 43(2-3):169-177; and Phagoo et al., (2001) J Pharmacol Exp Ther 298(1):77-85. Calcium channel (N-type) binding activity was examined using the method disclosed by Moresco et al., (1990) Neurobiol of Aging. 11(4):433-436. Cannabinoid receptor binding activity was examined using the method disclosed by Reggio et al., (1998) J Med Chem 41(26):5177-5187. Corticotrophin releasing factor (CRF₁) receptor binding activity was examined using the methods disclosed by Lewis et al., (2001) Proc Natl Acad Sci USA 98(13):7570-7575; and Sutton et al., (1995) Endocrinology 136(3):1097-1102. Estrogen receptors (including ERα and ERβ) were examined using the method disclosed by Obourn et al., (1993) Biochem 32:6229-6236. GABA receptor (including GABA_{A} and GABA_{B1A},) binding activity was examined using methods disclosed by Lewin et al., (1989) Mol Pharmacol 35:189-194; Maksay G. (1993) Eur J Pharmacol 246:255-260; Enna and Snyder, (1977) Mol Pharmacol. 13:442-453; Martinin et al., (1983) J Neurochem. 41:1183-1185; Damm et al., (1978) Res Comm Chem Pathol Pharmacol. 22:597-600; Speth et al., (1979) Life Sci. 24:351-357; Dubinsky et al., (2002) J Pharmacol Exp Ther. 303(2):777-790; and McLeod et al., (2002) Brain Res Mol Brain Res 104(2):203-209. Leptin receptor binding activity was examined using the method disclosed by Fong et al., (1998) Mol Pharmacol 53:234-240. Nicotinin acetylcholine receptor (including (α1, α4β2 and α7) binding activity was examined using the methods disclosed by Davila-Garcia et al., (1997) J Pharmacol Exp Ther 282:445-451 ; and Whiteaker et al., (2000) Br J Pharmacol. 131:729-739. Sodium chanel binding activity was examined using the method disclosed by Catterall et al., (1981) J Biol Chem. 256:8922-8927. Dopamine receptor binding activity was examined using the methods disclosed by Lewis et al., (2001) Proc Natl Acad Sci USA 98(13):7570-7575; and Sutton et al., (1995) Endocrinology 136(3):1097-1102. Glutamate receptor binding activity was examined using the method disclosed by Mutel et al., (2000) J Neurochem. 75(6):2590-2601. Tachykinin receptor binding activity was examined using.the method disclosed by Patacchini and Maggi, (1995) Arch Int Pharmacodyn. 329:161-184. Vanilloid receptor binding activity was examined using the methods disclosed by Szallasi et al., (1993) J Pharmacol Exp Ther. 267(2):728-733. Melatonin receptor binding activity was examined using the methods disclosed by Beresford et al., (1998) J Pharmacol Exp Ther 285(3):1239-1245; and Paul et al., (1999) J Pharmacol Exp Ther 290(1):334-340. Serotonin receptor binding activity was examined using the methods disclosed by Bohaus et a., (1995) Br J Pharmacol 115:622-628; and Saucier and Albert (1997) J. Neuorchem 68:1998-2011.

Binding on various receptors as described above were tested, and significant binding of (R)-(+)-verapamil hydrochloride and (S)-(-)-verapamil hydrochloride were observed in orexin receptor 1, orexin receptor 2, somatostatin receptor 2, sodium channel, L-type and N-type calcium channels, serotonin, melatonin and dopamine (D_{2L}) receptors (some data were not shown). Among them, binding of (R)-(+)-verapamil hydrochloride and (S)-(-)-verapamil hydrochloride with orexin receptor 1, orexin receptor 2, somatostatin receptor 2 are novel discovery of this application and have never been reported before.

Table 1 is a summary of the binding affinity results of (R)-(+)-verapamil hydrochloride and (S)-(-)-verapamil hydrochloride on orexin receptor 1, orexin receptor 2 and sodium channel. In the orexin assay, (R)-(+)-verapamil hydrochloride showed significant activity on orexin receptor 1 with an IC₅₀ value of 242 µM (Kᵢ = 170 µM and n_{H} = 1.31), and orexin receptor 2 with an IC₅₀ value of 83.7 µM (Kᵢ = 64 µM and n_{H} = 1.81). (S)-(-)-verapamil hydrochloride also showed significant activity on orexin receptor 1 with an IC₅₀ value of 69.8 µM (Kᵢ = 49 µM and n_{H} = 1.27), and orexin receptor 2 with an IC₅₀ value of 34.7 µM (Kᵢ = 26.5 µM and n_{H} = 1.33). For sodium channel, both (R)-(+)-verapamil hydrochloride and (S)-(-)-verapamil hydrochloride showed significant binding activity with an IC₅₀ value of 3.28 µM and 1.11 µM, respectively. Among them, binding of (R)-(+)-verapamil hydrochloride and (S)-(-)-verapamil hydrochloride with orexin receptor 1, orexin receptor 2 are novel discovery of this application and have never been reported before.

Further screening studies using SelectScreen^{®} Cell-based GPCR Profiling Service provided by Invitrogen (Madison, WI, USA) confirmed that both (R)-(+)-verapamil hydrochloride and (S)-(-)-verapamil hydrochloride act as antagonists to orexin receptor 1 and orexin receptor 2. Both agonist and antagonist assays were performed in accordance with the manfacturer's protocols, and (R)-(+)-verapamil hydrochloride and (S)-(-)-verapamil hydrochloride used in this assay were obtained from Syn-Tech Chem & Pharm Co. Ltd (Taiwan, R.O.C.). Resutls are provided in Table 2.

Table 3 summarizes the binding activity of (R)-(+)-verapamil hydrochloride and (S)-(-)-verapamil hydrochloride on L-type calcium channel (including atrial inotropy and ileum), somatostatin receptor 2 and dopamine (D_{2L}) receptor. In the somatostatin tissue assay, both (R)-(+)-verapamil hydrochloride and (S)-(-)-verapamil hydrochloride showed significant agonist activity with EC₅₀ value of 1.6 µM and 0.19 µM, respectively. In the calcium channel L-type, atrial inotropy tissue assay, both (R)-(+)-verapamil hydrochloride and (S)-(-)-verapamil hydrochloride showed significant antagonist activity with IC₅₀ value of 21.9 µM and 6.2 µM, respectively. In the calcium channel L-type, Ileum tissue assay, both (R)-(+)-verapamil hydrochloride and (S)-(-)-verapamil hydrochloride showed significant antagonist activity with IC₅₀ value of 0.04 µM and 6.8 nM, respectively. In the dopamine (D_{2L}) receptor assay, both (R)-(+)-verapamil hydrochloride and (S)-(-)-verapamil hydrochloride showed significant antagonist activity with IC₅₀ value of 5.97 µM and 1.47 µM, respectively. Among them, the agonist activities of (R)-(+)-verapamil hydrochloride and (S)-(-)-verapamil hydrochloride of somatostatin receptor 2, and the antagonist activities of (R)-(+)-verapamil hydrochloride and (S)-(-)-verapamil hydrochloride of dopamine (D_{2L}) receptor are novel discovery of this application and have never been reported before.

**Table 1 Binding Activity of (R)-(+)-verapamil hydrochloride and (S)-(-)-verapamil hydrochloride on orexin receptor 1 or 2 and Sodium Channel**

| **Receptor/Compound** | **Species** | **Conc. (µM)** | **% Inhibition** | **IC₅₀ (µM)** | **Kᵢ (µM)** | **n_{H}** |
|---|---|---|---|---|---|---|
| **Orexin receptor 1** | | | | | | |
| (R)-(+)-verapamil hydrochloride | Hum | 300 | 57 | 242 | 170 | 1.31 |
| (S)-(-)-verapamil hydrochloride | hum | 100 | 58 | 69.8 | 49 | 1.27 |
| **Orexin receptor 2** | | | | | | |
| (R)-(+)-verapamil hydrochloride | hum | 100 | 56 | 83.7 | 64 | 1.81 |
| (S)-(-)-verapamil hydrochloride | hum | 100 | 79 | 34.7 | 26.5 | 1.33 |
| **Sodium Channel, Site 2** | | | | | | |
| (R)-(+)-verapamil hydrochloride | rat | 10 | 72 | 3.28 | 2.99 | 0.938 |
| (S)-(-)-verapamil hydrochloride | rat | 3 | 75 | 1.11 | 1.02 | 1.1 |

| | | | | | | |
|---|---|---|---|---|---|---|
| Hum = human | | | | | | |

**Table 2 Agonist/Antagonist Assay Results of (R)-(+)-verapamil hydrochloride and (S)-(-)-verapamil hydrochloride on orexin receptor 1 or 2**

| **Receptor/Compound** | **Agonist EC₅₀ (µM)** | **Antagonist IC₅₀ (µM)** |
|---|---|---|
| **Orexin receptor 1** | | |
| (R)-(+)-verapamil hydrochloride | >300 | 136 |
| (S)-(-)-verapamil hydrochloride | >300 | 104 |
| **Orexin receptor 2** | | |
| (R)-(+)-verapamil hydrochloride | >300 | 198 |
| (S)-(-)-verapamil hydrochloride | >300 | 176 |

**Table 3 Binding Activity of (R)-(+)-verapamil hydrochloride and (S)-(-)-verapamil hydrochloride on L-type calcium channe, somatostatin receptor 2 and Dopamine D_{2L} receptor**

| **Receptor/Compound** | **Species** | **Conc.** | **AG. (%)** | **ANT. (%)** | **EC₅₀/IC₅₀** |
|---|---|---|---|---|---|
| **L Type Calcium Channel, Atrial Inotropy (Left artria)** | | | | | |
| (R)-(+)-verapamil hydrochloride | gp | 30 µM | 0 | 61 | 21.9 µM |
| (S)-(-)-verapamil hydrochloride | gp | 10 µM | 0 | 61 | 6.2 µM |
| **L Type Calcium Channel, Ileum** | | | | | |
| (R)-(+)-verapamil hydrochloride | gp | 0.1 µM | 0 | 83 | 0.04 µM |
| (S)-(-)-verapamil hydrochloride | gp | 10 nM | 0. | 66 | 6.8 nM |
| **Somatostatin receptor 2** | | | | | |
| (R)-(+)-verapamil hydrochloride | gp | 3 µM | 69 | ND | 1.6 µM |
| (S)-(-)-verapamil hydrochloride | gp | 0.3 µM | 61 | ND | 0.19 µM |
| **Dopamine D_{2L}** | | | | | |
| (R)-(+)-verapamil hydrochloride | hum | 10 µM | ND | 58 | 5.97 µM |
| (S)-(-)-verapamil hydrochloride | hum | 3 µM | ND | 66 | 1.47 µM |

| | | | | | |
|---|---|---|---|---|---|
| Hum = human, gp = guinea pig AG. = Agonist; ANT. = Antagonist; ND = not detected | | | | | |

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features. From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

## Claims

1. Verapamil for use in the treatment of a subject suffering from obesity or diabetes, wherein the verapamil is capable of antagonizing the function of orexin receptor 1 or orexin receptor 2 of the subject

2. The verapamil for use in the treatment of a subject suffering from obesity or diabetes as recited in claim 1, wherein the verapamil is any of (R)-(+)-verapamil, (S)-(-)-verapamil, a mixture of (R)-(+)-verapamil and (S)-(-)-verapamil, a pharmaceutically acceptable salt or solvate thereof, or a combination thereof

3. The verapamil for use in the treatment of a subject suffering from obesity or diabetes as recited in claim 2, wherein the verapamil is (R)-(+)-verapamil, a pharmaceutically acceptable salt or solvate thereof, or a combination thereof.

## Patentansprüche

1. Verapamil zur Anwendung in der Behandlung einer an Adipositas oder Diabetes erkrankten Person, wobei Verapamil bei der Person der Funktion des Orexin - Rezeptors 1 oder Orexin - Rezeptors 2 entgegenwirken kann.

2. Verapamil zur Anwendung in der Behandlung einer an Adipositas oder Diabetes erkrankten Person nach Anspruch 1, wobei Verapamil wahlweise (R)-(+)-Verapamil, (S)-(-)-Verapamil, eine Mischung aus (R)-(+)-Verapamil und (S)-(-)-Verapamil, ein pharmazeutisch verträgliches Salz oder Solvat davon oder eine Kombination daraus ist.

3. Verapamil zur Anwendung in der Behandlung einer an Adipositas oder Diabetes erkrankten Person nach Anspruch 2, wobei Verapamil (R)-(+)-Verapamil, ein pharmazeutisch verträgliches Salz oder Solvat davon oder eine Kombination daraus ist.

## Revendications

1. Vérapamil pour utilisation dans le traitement d'un sujet souffrant d'obésité ou de diabète, le vérapamil étant capable d'antagoniser la fonction du récepteur à orexine 1 ou du récepteur à orexine 2 du sujet.

2. Vérapamil pour utilisation dans le traitement d'un sujet souffrant d'obésité ou de diabète selon la revendication 1, le vérapamil étant l'un quelconque de (R)-(+)-vérapamil, (S)-(-)-vérapamil, un mélange de (R)-(+)-vérapamil et de (S)-(-)-vérapamil, un sel ou un solvate de celui-ci acceptable sur le plan pharmaceutique ou une combinaison de ceux-ci.

3. Vérapamil pour utilisation dans le traitement d'un sujet souffrant d'obésité ou de diabète selon la revendication 1, le vérapamil étant le (R)-(+)-vérapamil, un sel ou un solvate de celui-ci acceptable sur le plan pharmaceutique ou une combinaison de ceux-ci.
